# EUROPEAN PATENT SPECIFICATION

(11) **EP 3 310 246 B1**
(45) Date of publication and mention of the grant of the patent: **23.10.2019**
(21) Application number: 16753461.9
(22) Date of filing: 15.06.2016
(51) Int. Cl.: A61B 1/31, A61B 17/02

(54) **ROTATING ANOSCOPE**
ROTIERENDES ANOSKOP
ANUSCOPE ROTATIF

(30) Priority: 17.06.2015 IT UB20151475
(43) Date of publication of application: 25.04.2018
(73) Proprietor: Sias, Francesco, 09131 Cagliari (IT)
(72) Inventor: MANCA, Antonio, 27100 Pavia (IT)
(74) Representative: Crugnola, Pietro
(86) International application number: PCT/IB2016/053538
(87) International publication number: WO 2016/203395

(56) References cited:
- EP-A1- 2 692 282
- WO-A1-2006/033122
- US-A1- 2008 275 306

## Description

The present invention relates to an anoscope, which can be used in the proctologic field as a diagnostic and/or surgical instrument.

The anoscopes of known type usually comprise a cylindrical or cylindrical-conical portion - the so-called "speculum" - which can be handled by an operator through a handle and introduced (through the anus) in the final segment of the rectum of a patient. In the aforesaid speculum - which is hollow and open at the opposite ends - a dilator is inserted, which is longer than the speculum, is cylinder-shaped, has a substantial rounded end and is provided with a smooth surface. When the dilator is positioned inside the speculum and the latter is inserted into the rectum, the dilator acts by stretching the muscle wall of the rectum without damaging the mucosa thereof. After inserting the anoscope, the dilator is extracted while the speculum remains in seat. In this way, the lumen of the final segment of the rectum is temporarily dilated, thus making it possible to easily introduce other instruments and to carry out diagnostic and/or surgical maneuvers.

Since one of the known methods to surgically treat the hemorrhoids is to occlude the arterial branches that are afferent to the latter, namely to bind the wall of the aforesaid vessels by suture so as to narrow the lumen thereof and to stop the blood stream that is directed to the hemorrhoids, anoscopes have been made wherein an indentation is obtained in the lateral wall of the speculum. In that way, an operating window is made available, at which the rectal mucosa can protrude inside the speculum and through which suitable surgical instruments (such as pliers and suture needle holder) can come into contact with the mucosa near a preset operating site, for example near an arterial branch at the end of which a hemorrhoid has formed. If it is necessary to surgically treat several zones of rectal mucosa in a single patient and during a single intervention, the surgeon must rotate the speculum, in order to place the operating window near the zone of mucosa to be treated. First, the surgeon inserts the dilator, so as to stretch the rectal mucosa at the operating window and to avoid the mucosa becomes "pinched" in the window when the latter is moved. Subsequently, the surgeon holds the speculum and rotates the latter around the longitudinal axis thereof, according to a rotation angle that is such to bring the operating window in the desired position. After the operating window has been properly positioned, the surgeon can extract the dilator and treat the portion of rectal mucosa protruding through the operating window. However, this procedure substantially prolongs the time of intervention and thus causes a significant discomfort in the patient undergoing the surgical treatment.

The international application publication no. WO2004/021874 (in the name of the Applicant Dr. Francesco Sias) describes a rotating operating anoscope, comprising a fixed portion, one proximal end of which is provided with a handle that can be handled by an operator, and a mobile portion, which is shapingly coupled with the fixed portion. The mobile portion can rotate inside the fixed portion and is suitable, in use, to receive a dilator. A distal end of the mobile portion protrudes, by a preset length, from a corresponding distal opening of the fixed portion and an indentation, namely an operating window, is obtained in this distal end.

The aforesaid anoscope enables the operating window to be positioned successively at different zones of rectal mucosa by acting only on a part of the anoscope and without having to rotate the whole instrument. Moreover, the dimensions of the operating window are such to make accessible only the zones of rectal mucosa that are actually interested by the surgical treatment, thus avoiding that excessive portions of mucosa protrude through the operating window and obstruct the surgical field. The anoscope of WO2004/021874 is further provided with angular positioning means, arranged for adjusting the relative angular position of the mobile portion inside the fixed portion at preset reciprocal angular positions. Particularly, the angular positioning means comprises an adjustment ring, which controls the 360° rotation of the mobile portion and allows the latter to block at six alternative positions. The six alternative positions correspond with the same number of positons of the operating window with respect to the internal wall of the rectum and, more precisely, they correspond to the positions of six arterial branches being treatable by means of hemorrhoidal dearterialization with transanal anopexy. The latter is a particularly effective surgical technique (conceived by the Applicant), which is based on the constant presence (along the rectum wall) of six terminal arterial branches coming from the superior hemorrhoidal artery and at the end of which typically the hemorrhoids form. By placing a patient in a prone position, so as to place the anal opening frontally, the six arterial branches are positioned - in a virtual clock face - at 1, 3, 5, 7, 9 and 11 o'clock, respectively. Therefore, by using the anoscope of WO2004/021874, a surgeon can easily place the window at the arterial branch, at the end of which the hemorrhoid is formed, and bind the aforesaid branch by acting upstream the hemorrhoids.

A drawback of the anoscope disclosed in WO2004/021874 consists in the fact that it is possible to rotate the ring - and therefore to rotate the operating window - also when the dilator is not inserted inside the mobile portion. This means that, if the ring and consequently the operating window are accidentally rotated in the absence of the dilator after surgically treating a zone of rectal mucosa - and therefore in a step in which the aforesaid zone of rectal mucosa protrudes inside the anoscope through the operating window - there is a substantial risk that the stitches are dragged by the operating window, with a consequent damage for the patient.

US 2008/0275306 A1 discloses a composite anoscope for ano-rectal diagnostic and surgery, wherein the anoscope also comprises an internal component, to be inserted therein, and a suitably shorter external component ending with a conic frustum mantle, wherein the anoscope is to be inserted. The three components are conic frustum shaped hollow bodies having circular cross-section. The internal component, when inserted in the anoscope, forms therewith a single body having a compact and smooth external surface and an ogival tip. The external component is provided close to its mouth with slotted wings, to be fixed to the perianal skin for securing the whole composite anoscope during the diagnostic and surgical operations.

An object of the present invention is to improve the anoscopes that can be used in the surgical and diagnostic field.

Another object is to provide an anoscope, of the type comprising a mobile portion that rotates inside a fixed portion and provided with a window that is obtained in the mobile portion, in which it is not possible to rotate the mobile portion, and thus the window, when the dilator is not inserted inside the anoscope.

According to the invention, an anoscope is provided, as defined in claim 1. An anoscope is made available that comprises a mobile portion that rotates inside a fixed portion and is provided with a window that is obtained in the mobile portion, in which it is not possible to rotate the mobile portion, and thus the window, when the dilator is not inserted inside the anoscope.

As it will be explained in more detail in the following, this is made possible by the presence of positioning and locking means, which is shaped in such a manner as to prevent the mobile portion from coaxially rotating inside the fixed portion when the internal cavity of the anoscope, which is defined by the mobile portion, is not occupied by the dilator.

The invention will be better understood with reference to the attached drawings, which show some exemplary and non-limiting embodiments thereof, in which:
Figure 1 is an exploded perspective view, showing an anoscope according to the invention,
comprising a fixed portion, a mobile portion and a dilator;
Figure 2 is a side sectional view of the mobile portion of Figure 1;
Figure 3 is a front view of the proximal end of the mobile portion of Figure 1;
Figure 4 is a front view of the proximal end of the fixed portion of Figure 1;
Figure 5 is a schematic side sectional view of the anoscope of Figure 1, showing the mobile portion housed in the fixed portion and the dilator inserted in the mobile portion.

In the present description and in the attached claims, the adjective "proximal" defines a part, segment or end of the anoscope 1, which, in use, faces an operator (for example, a surgeon) holding and/or using the anoscope. Consequently, the adjective "distal" defines a part, segment or end of the anoscope 1, which, in use, faces a direction opposite to the operator holding and/or using the anoscope.

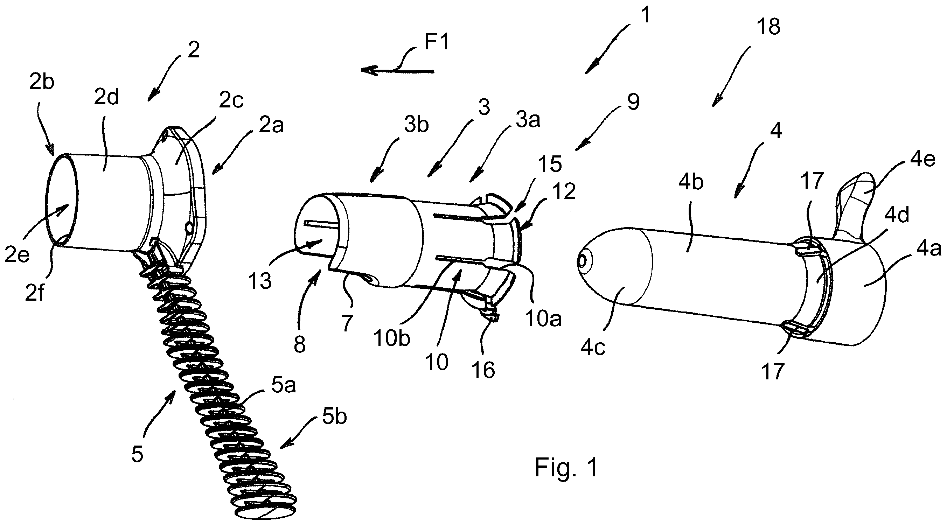

Figures 1 to 5 show an anoscope 1, comprising a fixed portion 2, a mobile portion 3 and a dilator 4. The fixed portion 2, the mobile portion 3 and the dilator 4 are made of a sterilizable polymeric material, for example polypropylene, or of another material that is suitable for medical use.

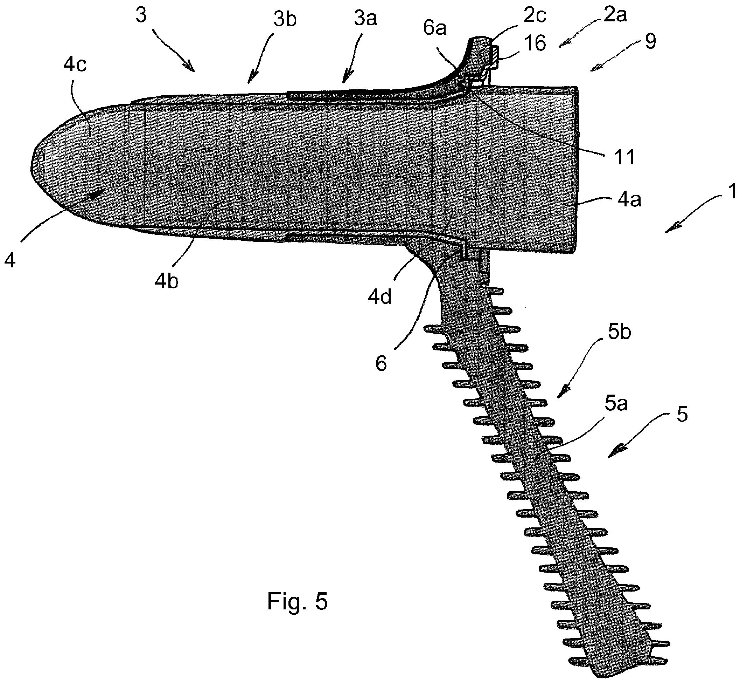

The fixed portion 2 and the mobile portion 3, which are shapingly coupled, are hollow and open at the opposite ends. In use, namely when the anoscope 1 is assembled (Figure 5), the mobile portion 3 is received inside the fixed portion 2, while the dilator 4 can be alternatively inserted in, or extracted from, the mobile portion 3. Owings to the positioning and locking means that is provided by the invention (the structure of which and the operation of which will be described in detail in the following), the mobile portion 3 can coaxially rotate by 360° with respect to the fixed portion 2 only when the dilator 4 is inserted in the mobile portion 3.

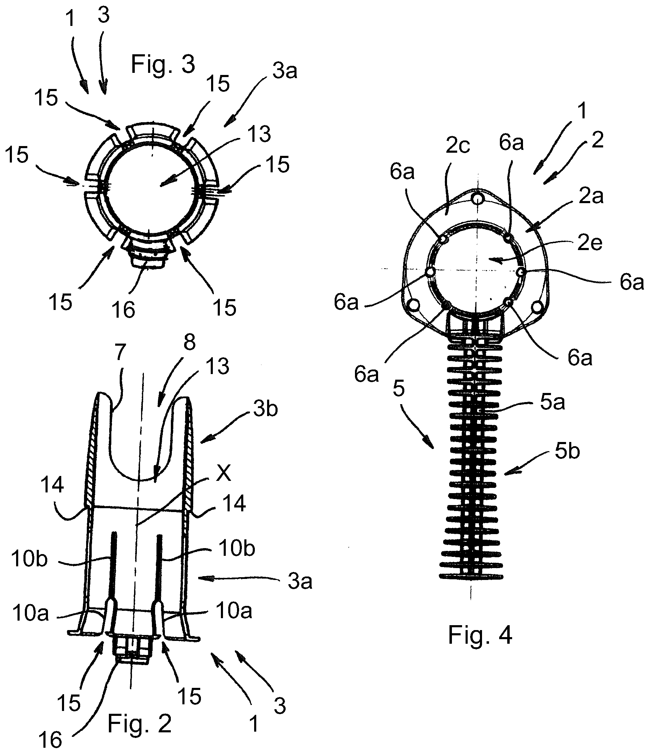

The fixed portion 2 (Figure 1; Figure 4; Figure 5) comprises a proximal end 2a and a distal end 2b, the latter being delimited by an edge 2f. The two ends 2a, 2b are open and reciprocally opposite. The proximal end 2a, in use, is the end of the fixed portion 2 - and thus of the anoscope 1 - facing towards an operator that uses the anoscope. Going from the proximal end 2a to the distal end 2b, the fixed portion 2 comprises a proximal segment 2c, which is approximately shaped as a frustum of cone, and a distal segment 2d, which is approximately cylinder-shaped. The proximal segment 2c and the distal segment 2d are both hollow, are reciprocally connected and define as a whole a housing 2e, which is arranged for receiving the mobile portion 3 and extends parallel to a longitudinal axis X (Figure 2) of the anoscope 1.

The fixed portion 2 comprises a handle 5, provided with a shape that is substantially ergonomic and suitable for allowing the operator to easily hold and handle the anoscope 1. Particularly, the handle 5 has a substantially segmented shape (Figure 1; Figure 4; Figure 5) and comprises a central element 5a from which a plurality of approximately discoidal protrusions 5b lead.

In one embodiment that is not shown, the handle 5 does not have the segmented structure that is shown in the Figures and it is internally hollow. In this way, inside the handle 5 a coaxial optical guide can be made, which is suitable for housing a lighting device (of known type) in order to light, in use, the operating field (namely the internal cavity of the anoscope 1).

The mobile portion 3 (Figure 1; Figure 2; Figure 3; Figure 5) comprises a rotation segment 3a and an operating segment 3b, which are hollow and connected reciprocally. The rotation segment 3a and the operating segment 3b define as a whole a longitudinal cavity 13 - namely the internal cavity - of the anoscope 1, which extends parallel to the longitudinal axis X of the latter. Therefore, the dilator 4 can be alternatively inserted in, or extracted from, the longitudinal cavity 13.

The rotation segment 3a is approximately conical-cylindrical, while the operating segment 3b is distally tapered and approximately shaped as a frustum of cone. The rotation segment 3a corresponds to the proximal part of the mobile portion 3, while the operating segment 3b corresponds to the distal part of the mobile portion 3.

At one end of the operating segment 3b that is opposite to the rotation segment 3a, a U-shaped indentation 7 is obtained, the concavity of which faces the direction opposite to the rotation segment 3a. The indentation 7 defines a window 8 that, in use, makes accessible to the operator a portion of rectal mucosa to be surgically treated or to be subjected to diagnosis. Since the external diameter of the rotation segment 3a is substantially smaller than the external diameter of the operating segment 3b, in the zone where the two segments join an abutment zone 14 is defined (Figure 2), which is ring shaped.

When the anoscope 1 is assembled, the mobile portion 3 is positioned in such a manner that the operating segment 3b faces the proximal end 2a of the fixed portion 2. Then the mobile portion 3 is moved along a direction shown by the arrow F1 (Figure 1), by inserting the operating segment 3b and the rotation segment 3a in the housing 2e and continuing until the operating segment 3b exits the distal end 2b of the fixed portion 2. This is made possible by the fact that the material which the mobile portion 3 is made of is substantially reversibly deformable during the insertion step in the fixed portion 2. After the operating segment 3b exits the distal end 2b, the abutment zone 14 is abutted against the adjacent edge 2f of the fixed portion 2.

In the mobile portion 3 a plurality of grooves 10 is obtained. Each groove 10 is obtained in the thickness of the lateral wall of the rotation segment 3a and comprises a proximal segment 10a, which is approximately U-shaped and has a concavity facing the direction opposite to the operating segment 3b, and a distal segment 10b, which is straight and leads from the convexity of the proximal segment 10a.

The proximal segment 10a of each groove continues with a notch 15, which is obtained in a flange portion 12, which peripherally protrudes from the free (proximal) end of the rotation segment 3a and is arranged substantially transversally to the latter. The distal segments 10b of the grooves 10 allow the operator to temporarily reduce the transversal diameter of the mobile portion 3 (and particularly the transversal diameter of the rotation segment 3a), when the latter is inserted in the fixed portion 2 (as previously described), so as to properly position the mobile portion 3 in the housing 2e.

A slider 16 is fixed to the flange portion 12, in a position of the latter that is comprised between two consecutive notches 15. The slider 16 is aligned with the window 8 (Figure 1; Figure 2) and thus signals the position of the latter, in use, when the mobile portion 3 is rotated inside the fixed portion 2 (namely, when the dilator 4 is inserted in the longitudinal cavity 13). In the assembled anoscope 1, the slider 16 is positioned at the proximal end 2a (Figure 5) and can slide along the latter, following the rotation of the mobile portion 3, when the dilator 4 is inserted in the longitudinal cavity 13.

The anoscope 1 is provided with positioning and locking means 9, enabling the mobile portion 3 to be placed in a preset angular position with respect to the fixed portion 2 and to become blocked in the above-mentioned angular position when the dilator 4 is not inserted in the longitudinal channel 13. The positioning and locking means 9 comprises a plurality of recesses 6a and a stop 11.

The recesses 6a are in number of six (Figure 4) and are made at a shoulder 6 (Figure 5) obtained in an internal portion of the proximal end 2a of the fixed portion 2. Each recess 6a is approximately hemispheric and is shapingly coupled with the stop 11.

The stop 11 is approximately hemispheric and is made in the mobile part 3 near the slider 16. More precisely, the stop 11 is made in the zone of the flange portion 12 which the slider 16 is fixed to and which - in the assembled anoscope 1 - faces the proximal end 2a of the fixed portion 2, as shown in Figure 5.

It should be noted that, in Figure 5, the recess 6a is not shown in the exact position where it should be, but in a position that is suitable for enabling the recess 6a to be schematically represented in a side sectional view (such as the one of Figure 5).

Actually, in the anoscope 1 according to the invention, by matching the proximal end 2a to the (circular) outline of a virtual clock face, the positions of the six recesses 6a substantially correspond to 1, 3, 5, 7, 9, 11 o'clock (Figure 4), namely to the positions of the six terminal arterial branches of the superior hemorrhoidal artery. The recesses 6a are therefore obtained in preset positions of the proximal end 2a. This configuration of the recesses 6a enables the anoscope 1 to be used in a way similar to the rotating operating anoscope being disclosed in WO2004/021874, namely to surgically treat the hemorrhoids, which can form at the end of the aforesaid arterial branches, by using the surgical technique (hemorrhoidal dearterialization with transanal anopexy) conceived by the Applicant.

When the anoscope 1 is assembled, the mobile portion 3 is partially inserted (along the direction F1) in the housing 2e of the fixed portion 2 and it is rotated with respect to the longitudinal axis X (of the anoscope 1) in such a way that the stop 11 is placed at any of the six recesses 6a. This can be obtained by looking at the position of the slider 16 (that is aligned with the window 8) while the mobile portion 3 rotates and by stopping the rotation of the mobile portion 3 when the slider 16 reaches the corresponding position at 1, 3, 5, 7, 9 or 11 o'clock. At this point, the mobile portion 3 is fully inserted in the housing 2e, so as to insert the stop 11 in the corresponding recess 6a. Since the recesses 6a and the stop 11 are shapingly coupled, the stop 11 and the corresponding recess 6a reciprocally engage in such a way as to block the coaxial rotation of the mobile portion 3 inside the fixed portion 2. Therefore, the positioning and locking means 9 effectively enables the mobile portion 3 to be placed, as well as be blocked, at a preset angular position with respect to the fixed portion 2. When the mobile portion 3 is so blocked, the window 8 is similarly positioned at the corresponding hour (1, 3, 5, 7, 9 or 11) of the virtual clock face. Since the slider 16 is aligned with the window 8, the hour (namely, the position) at which the window 8 is positioned is clearly shown by the position of the slider 16 with respect to the proximal end 2a of the fixed portion 2.

In order to unlock the mobile portion 3 and allow the latter to rotate by 360° in the housing 2e, it is necessary to insert the dilator 4 in the longitudinal cavity 13.

The latter comprises (Figure 1; Figure 5) a proximal portion 4a, an intermediate portion 4b, a distal portion 4c, a connecting portion 4d and a tongue portion 4e. The intermediate portion 4b is elongated, cylindrical and it forms the body of the dilator 4. The intermediate portion 4b is interposed between the connecting portion 4d and the distal portion 4c, which corresponds to the tip of the dilator 4 and it is tapered and/or rounded. The proximal portion 4a is cylinder-shaped, it is hollow internally and it laterally elongates to form the tongue portion 4e, which protrudes slantwise from a zone of the proximal portion 4a opposite to the intermediate portion 4b. The tongue portion 4e has a curvilinear profile and is provided with an ergonomic shape, which is suitable for allowing the handling thereof by the operator. Between the proximal portion 4a and the intermediate portion 4b the connecting portion 4d is interposed, which is approximately frusto-conically shaped and the major base of which is adjacent to the proximal portion 4a. As shown in Figure 5, the connecting portion 4d and the intermediate portion 4b are shapingly coupled with the longitudinal cavity 13 that is defined inside the mobile portion 3. Moreover, the total length of the connecting portion 4d and the intermediate portion 4b is such as to allow the distal portion 4c alone to longitudinally protrude outside the operating segment 3b.

On the external surface of the connecting portion 4d a plurality of jutting elements 17 are obtained that are shapingly coupled with the grooves 10. More precisely, the jutting elements 17 are shapingly coupled with the proximal segments 10a of the grooves 10 and therefore they are provided with an approximately U-shaped profile (having a convexity faced towards the distal portion 4c). In the version of the anoscope 1 that is shown in the Figures, the jutting elements 17 are three (only two of which are shown in Figure 1) and are reciprocally staggered by 120°. In embodiments that are not shown, the jutting elements 17 can be in a number lesser than three, or greater than three (up to six maximum).

In order to complete the assemblage of the anoscope 1, after inserting and blocking the mobile portion 3 inside the fixed portion 2 (as previously disclosed), the dilator 4 is inserted in the longitudinal cavity 13 along the direction F1, in such a way that each jutting element 17 reaches and fully engages the proximal segment 10a of a corresponding groove 10. In this way, it is possible to unlock the mobile portion 3 by acting on the tongue portion 4e, so as to rotate the dilator 4 clockwise or anticlockwise. In fact, since each jutting element 17 engages a corresponding proximal segment 10a, by rotating the dilator 4 the stop 11 is forced to disengage the corresponding recess 6a, so as to exit the latter. The mobile portion 3 can then rotate, following the rotation of the dilator 4, and the stop 11 can slide in contact with the shoulder 6 of the proximal end 2a until reaching a next recess 6a (and therefore a following position for the window 8) and engaging the latter. In this step, if the operator stops rotating the dilator 4 (and therefore the mobile portion 3) and extracts the dilator 4 from the longitudinal cavity 13 - by moving the dilator 4 along a direction (that is not shown) that is parallel and opposite to the direction F1 - the mobile portion will be blocked, owing to the stop 11 that engages the recess 6a, and the window 8 will be consequently positioned (and blocked) at a desired point of intervention. If, on the contrary, the operator continues with rotating the dilator 4 (and therefore the mobile portion 3), the stop 11 will disengage the recess 6a, it will exit the latter and it will continue with sliding in contact with the shoulder 6 until reaching the following recess 6a. The jutting elements 17 and the grooves 10, and particularly the proximal segments 10a of these latter, as a whole constitute unlocking means 18, which is made in the mobile portion 3 and the dilator 4. In use, the unlocking means 18 acts in a complementary way with the positioning and locking means, allowing the anoscope 1 to operate in an efficient manner. One possible mode of use of the anoscope 1 according to the invention is disclosed in the following, for a merely exemplary purpose.

The operator, by holding the anoscope 1 complete with the dilator 4 (that is inserted in the longitudinal channel 13) through the handle 5, orients the anoscope 1 in such a way that the handle 5 is parallel to the intergluteal cleft of a patient to be treated and the operating segment 3b is positioned near the patient's anal opening. The anoscope 1 can then be inserted in the final segment of the rectum, owing to the dilator 4 that dilates the intestinal lumen and stretches the intestinal mucosa, preceding and facilitating the access of the mobile portion 3 and the fixed portion 2. Once positioned the anoscope 1 in the final segment of the rectum, the operator can grip the tongue portion 4e and, by acting on the latter, can unlock and clockwise or anticlockwise rotate the mobile portion 3. This is made possible by the unlocking means 18, namely by the jutting elements 17 of the dilator 4 and by the proximal segments 10a of the grooves 10, reciprocally engaging and enabling the stop 11 to be released from the respective recess 6a. The operator rotates the mobile portion 3 until bringing the window 8 in the desired position (that is shown by the position of the slider 16) and extracts the dilator 4 from the longitudinal cavity 13. In this way, a portion of rectal mucosa to be surgically treated (or to be examined for a diagnostic purpose) is made accessible by the operator and it is not possible anymore to rotate the mobile portion 3 with respect to the fixed portion 2, since the stop 11 engages a corresponding recess 6a. Therefore, after surgically treating (or examining) the portion of rectal mucosa that is positioned at the window 8, in order to rotate the mobile portion 3 and to change the position of the window 8, the operator necessarily will have to insert the dilator 4 in the longitudinal cavity 13, so as to disengage the stop 11 from the recess 6a and to unlock the mobile portion 3. In this way, the risk is substantially avoided that the mobile portion 3 is accidentally rotated in the absence of the dilator 4 and the suture stitches are dragged by the window 8, with a consequent damage for the patient.

The anoscope 1 can be effectively used, in addition to surgically treat the hemorrhoids, also for: carrying out elastic ligations, cryotherapy, resection of intestinal polyps, carrying out sclerosing injections, trans-anal diagnosis and therapy in general.

Variations and/or additions are also possible to what disclosed above and/or to what shown in the attached drawings.

## Claims

1. Anoscope (1), comprising a fixed portion (2) that is hollow and open at the opposite ends, a mobile portion (3) that is hollow and open at the opposite ends, said mobile portion (3) being shapingly coupled with said fixed portion (2) and being arranged for rotating coaxially inside said fixed portion (2), a window (8) that is obtained in said mobile portion (3) and is arranged for making accessible a portion of rectal mucosa, a dilator (4) that can be alternatively inserted in, or extracted from, a longitudinal cavity (13) of said anoscope (1), said longitudinal cavity (13) being comprised in said mobile portion, positioning and locking means (9) arranged for preventing said mobile portion (3) from rotating coaxially inside said fixed portion (2) when said dilator (4) is not inserted in said longitudinal cavity (13), wherein said positioning and locking means (9) comprises a stop (11) and a plurality of recesses (6a), said stop (11) being suitable for engaging, in use, one of said recesses (6a) so as to prevent said mobile portion (3) from rotating coaxially inside said fixed portion (2), said anoscope (1) comprising unlocking means (18) that is made in said mobile portion (3) and in said dilator (4), said unlocking means (18) being so shaped as to enable, in use, to disengage said stop (11) from said recess (6a) when said dilator (4) is inserted in said longitudinal cavity (13),
said anoscope (1) being **characterized in that**
said unlocking means (18) comprises a plurality of jutting elements (17) that are made in said dilator (4) and a plurality of grooves (10) that are obtained in said mobile portion (3), said jutting elements (17) being shapingly coupled with said grooves (10) and being arranged for engaging said grooves (10) when said dilator (4) is inserted in said longitudinal cavity (13), wherein, when said jutting elements (17) engage said grooves (10), said dilator (4) can be rotated inside said longitudinal cavity (13) in such a manner as to force said stop (11) to disengage said recess (6a) and enable said mobile portion (3) to rotate coaxially inside said fixed portion (2).

2. Anoscope (1) according to claim 1, wherein said stop (11) is made in said mobile portion (3) and said recesses (6a) are obtained in said fixed portion (2).

3. Anoscope (1) according to claim 2, wherein said stop (11) is made near to a slider (16) that is fixed to a proximal part of said mobile portion (3) and said recesses (6a) are obtained in a proximal end (2a) of said fixed portion (2).

4. Anoscope (1) according to claim 3, wherein said stop (11) is made in a flange portion (12) that is comprised in said proximal part of said mobile portion (2).

5. Anoscope (1) according to claim 4, wherein said stop (11) is made in a zone of said flange portion (12) that faces, in use, said proximal end (2a) of said fixed portion (2).

6. Anoscope (1) according to any one of claims 1 to 5, wherein said jutting elements (17) are shapingly coupled with corresponding proximal segments (10a) of said grooves (10).

7. Anoscope (1) according to any one of claims 3 to 6, wherein said recesses (6a) are obtained in preset positions of said proximal end (2a), so as to enable said mobile portion (3) and said window (8) to be positioned and locked in a plurality of preset and alternative angular positions with respect to said fixed portion (2).

## Patentansprüche

1. Anoskop (1), das Folgendes aufweist, nämlich einen festes Teil (2), das hohl ist und an den gegenüberliegenden Enden offen ist, und ein bewegliches Teil (3), das hohl ist und an den gegenüberliegenden Enden offen ist, wobei das bewegliche Teil (3) mit dem festen Teil (2) formgekoppelt ist und für eine koaxiale Rotation im Inneren der festen Position (2) eingerichtet ist, ein Fenster (8), das in dem beweglichen Teil (3) vorgesehen ist und eingerichtet ist, um einen Teil der rektalen Mukosa zugänglich zu machen, einen Dilator (4), der alternativ in eine längliche Kavität (13) des Anoskops (1) eingebracht oder aus dieser herausgenommen werden kann, wobei die längliche Kavität (13) in dem beweglichen Teil vorgesehen ist, ein Positionierungs- und Verriegelungsmittel (9), das eingerichtet ist, um zu verhindern, dass das bewegliche Teil (3) im Inneren des festen Teils (2) koaxial rotiert, wenn der Dilator (4) nicht in der länglichen Kavität (13) eingebracht ist, wobei das Positionierungs- und Verriegelungsmittel (9) eine Sperre (11) und eine Vielzahl von Ausnehmungen (6a) aufweist, wobei die Sperre (11) für ein Eingreifen, bei Benutzung, in eine der Ausnehmungen (6a) geeignet ist, um zu verhindern, dass das bewegliche Teil (3) im Inneren des festen Teils (2) koaxial rotiert, wobei das Anoskop (1) ein Entriegelungsmittel (18) aufweist, das in dem beweglichen Teil (3) und in dem Dilator (4) vorgesehen ist, wobei das Entriegelungsmittel (18) derart ausgestaltet ist, dass es bei Benutzung die Sperre (11) von der Ausnehmung (6a) löst, wenn der Dilator (4) in die längliche Kavität (13) eingebracht wird,
wobei das Anoskop (1) **dadurch gekennzeichnet ist, dass**
das Entriegelungsmittel (18) eine Vielzahl von herausragenden Elementen (17) aufweist, die in dem Dilator (4) vorgesehen ist, und eine Vielzahl von Schlitzen (10), die in dem beweglichen Teil (3) vorgesehen ist, wobei die herausragenden Elemente (17) mit den Schlitzen (10) formgekoppelt sind und eingerichtet sind, um in die Schlitze (10) einzugreifen, wenn der Dilator (4) in die längliche Kavität (13) eingebracht wird, wobei, wenn die herausragenden Elemente (17) in die Schlitzen (10) eingreifen, der Dilator (4) im Inneren der länglichen Kavität (13) derart rotiert werden kann, dass er die Sperre (11) dazu veranlasst, die Ausnehmung (6a) zu lösen und es dem beweglichen Teil (3) ermöglicht, im Inneren des festen Teils (2) koaxial zu rotieren.

2. Anoskop (1) gemäß Anspruch 1, wobei die Sperre (11) in dem beweglichen Teil (3) vorgesehen ist und die Ausnehmungen (6a) in dem festen Teil (2) vorgesehen sind.

3. Anoskop (1) gemäß Anspruch 2, wobei die Sperre (11) in der Nähe eines Schiebers (16) vorgesehen ist, der an einem proximalen Teil des beweglichen Teils (3) befestigt ist und die Ausnehmungen (6a) in einem proximalen Ende (2a) des festen Teils (2) vorgesehen sind.

4. Anoskop (1) gemäß Anspruch 3, wobei die Sperre (11) in einem Flanschteil (12) vorgesehen ist, das in dem proximalen Teil des beweglichen Teils (2) vorgesehen ist.

5. Anoskop (1) gemäß Anspruch 4, wobei die Sperre (11) in einem Bereich des Flanschteils (12) vorgesehen ist, der, in Benutzung, dem proximalen Ende (2a) des festen Teils (2) zugewandt ist.

6. Anoskop (1) gemäß einem der Ansprüche 1 bis 5, wobei die herausragenden Elemente (17) mit korrespondierenden Segmenten (10a) der Schlitze (10) formgekoppelt sind.

7. Anoskop (1) gemäß einem der Ansprüche 3 bis 6, wobei die Ausnehmungen (6a) in vorgegebenen Positionen des proximalen Endes (2a) derart vorgesehen sind, dass das bewegliche Teil (3) und das Fenster (8) in einer Vielzahl von vorgegebenen und alternativen winkligen Positionen in Bezug auf das feste Teil (2) positioniert und verriegelt werden können.

## Revendications

1. Anuscope (1) comprenant une partie fixe (2) qui est creuse et ouverte aux extrémités opposées, une partie mobile (3) qui est creuse et ouverte aux extrémités opposées, ladite partie mobile (3) étant reliée par complémentarité de forme à ladite partie fixe (2) et étant agencée pour tourner coaxialement à l'intérieur de ladite partie fixe (2), une fenêtre (8) qui est obtenue dans ladite partie mobile (3) et qui est agencée pour rendre accessible une partie de la muqueuse rectale, un dilatateur (4) qui peut être alternativement inséré dans ou extrait d'une cavité longitudinale (13) dudit anuscope (1), ladite cavité longitudinale (13) étant comprise dans ladite partie mobile, des moyens de positionnement et de verrouillage (9) agencés pour empêcher ladite partie mobile (3) de tourner coaxialement à l'intérieur de ladite partie fixe (2) quand ledit dilatateur (4) n'est pas inséré dans ladite cavité longitudinale (13), dans lequel lesdits moyens de positionnement et de verrouillage (9) comprennent une butée (11) et une pluralité d'évidements (6a), ladite butée (11) étant apte à venir en prise, lors de l'utilisation, avec l'un desdits évidements (6a) de manière à empêcher ladite partie mobile (3) de tourner coaxialement à l'intérieur de ladite partie fixe (2), ledit anuscope (1) comprenant des moyens de déverrouillage (18) qui sont réalisés dans ladite partie mobile (3) et dans ledit dilatateur (4), lesdits moyens de déverrouillage (18) étant formés de manière à permettre, lors de l'utilisation, de supprimer la prise entre ladite butée (11) et ledit évidement (6a) quand ledit dilatateur (4) est inséré dans ladite cavité longitudinale (13),
ledit anuscope (1) étant **caractérisé en ce que** lesdits moyens de déverrouillage (18) comprennent une pluralité d'éléments saillants (17) qui sont réalisés dans ledit dilatateur (4) et une pluralité de rainures (10) qui sont obtenues dans ladite partie mobile (3), lesdits éléments saillants (17) étant reliés par complémentarité de forme auxdites rainures (10) et étant agencés pour venir en prise avec les rainures (10) quand ledit dilatateur (4) est inséré dans ladite cavité longitudinale (13), dans lequel, quand lesdits éléments saillants (17) sont en prise avec lesdites rainures (10), ledit dilatateur (4) peut être tourné à l'intérieur de ladite cavité longitudinale (13) de manière à forcer ladite butée (11) à supprimer la prise avec ledit évidement (6a) et à permettre à ladite partie mobile (3) de tourner coaxialement à l'intérieur de ladite partie fixe (2).

2. Anuscope (1) selon la revendication 1, dans lequel ladite butée (11) est réalisée dans ladite partie mobile (3), et lesdits évidements (6a) sont obtenus dans ladite partie fixe (2).

3. Anuscope (1) selon la revendication 2, dans lequel ladite butée (11) est réalisée près d'un coulisseau (16) qui est fixé à une partie proximale de ladite partie mobile (3), et lesdits évidements (6a) sont obtenus dans une extrémité proximale (2a) de ladite partie fixe (2).

4. Anuscope (1) selon la revendication 3, dans lequel ladite butée (11) est réalisée dans une partie formant collerette (12) qui est comprise dans ladite partie proximale de ladite partie mobile (2).

5. Anuscope (1) selon la revendication 4, dans lequel ladite butée (11) est réalisée dans une zone de ladite partie formant collerette (12) qui fait face, lors de l'utilisation, à ladite extrémité proximale (2a) de ladite partie fixe (2).

6. Anuscope (1) selon l'une quelconque des revendications 1 à 5, dans lequel lesdits éléments saillants (17) sont reliés par complémentarité de forme à des segments proximaux correspondants (10a) desdites rainures (10).

7. Anuscope (1) selon l'une quelconque des revendications 3 à 6, dans lequel lesdits évidements (6a) sont obtenus dans des positions préréglées de ladite extrémité proximale (2a) de manière à permettre à ladite partie mobile (3) et à ladite fenêtre (8) d'être positionnées et verrouillées dans une pluralité de positions angulaires préréglées et alternatives par rapport à ladite partie fixe (2).
